(19) Europäisches Patentamt
European Patent Office
Office européen des brevets

(11) **EP 4 435 717 A1**

(12) **EUROPEAN PATENT APPLICATION**

(43) Date of publication:
**25.09.2024  Bulletin 2024/39**

(21) Application number: **23164102.8**

(22) Date of filing: **24.03.2023**

(51) International Patent Classification (IPC):
**G06T 7/11** (2017.01)     **G06T 7/73** (2017.01)
**G16B 40/10** (2019.01)     **G06T 7/00** (2017.01)

(52) Cooperative Patent Classification (CPC):
**G06T 7/75; G06T 7/0012; G06T 7/11; G16B 40/10;**
G06T 2207/10056; G06T 2207/10064;
G06T 2207/30004; G06T 2207/30024;
G06T 2207/30072

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **QIAGEN GmbH
40724 Hilden (DE)**

(72) Inventor: **KRANZ, Klaus
40724 Hilden (DE)**

(74) Representative: **König Szynka Tilmann von
Renesse
Patentanwälte Partnerschaft mbB Düsseldorf
Mönchenwerther Straße 11
40545 Düsseldorf (DE)**

(54)     **METHOD FOR PROVIDING AN ANALYSIS OF PARTITION IMAGE DATA**

(57)     The invention relates to a method (200) for providing an analysis of partition image data (142), in particular for providing an analysis of a plurality of partition states of at least one microfluidic well (134) within a digital polymerase chain reaction analysis. The method comprising the steps
- receiving the partition image data which is indicating an optical appearance of a plurality of partitions (132) that are arranged in a preexisting grid structure (365);
- calculating a matrix correlation function R(x,y) for each point (x,y) of the at least one microfluidic well depicted within the partition image data, wherein the matrix correlation function R(x,y) indicates a correlation between a predefined template partition matrix T(x',y') and the at least one depicted microfluidic well;
- calculating a set of assumed grid point locations (360) within the partition image data based on a predefined number of expected grid points and on the calculated matrix correlation function R(x,y);
- detecting a respective partition state based on an optical appearance of the calculated set of assumed grid point locations within the partition image data for each partition of the at least one microfluidic well; and
- providing analysis information (125) based on the detected partition states.

Fig. 5

**Description**

FIELD OF THE INVENTION

The invention relates to a method for providing an analysis of partition image data, in particular for providing an analysis of a plurality of partition states of at least one microfluidic well within a digital polymerase chain reaction analysis. Furthermore, the invention relates to a system for providing a molecular analysis of a fluid sample, in particular for providing a digital polymerase chain reaction analysis, and to a computer program for operating a system comprising program code means for causing a computer to carry out said method.

BACKGROUND OF THE INVENTION

The analysis of partition image data is well known in the field of molecular analysis. In particular, methods in the field of polymerase chain reaction (PCR) analysis are often based on visual findings.

PCR methods usually rely on thermal cycling, which involves a thermal treatment of reactants permitting different temperature-dependent reactions. Digital PCR describes a PCR analysis where a large number of separate aliquots located in respective partitions is analyzed in order to detect after the thermal treatment, whether a molecule of target DNA was present in the respective aliquot or not. In view of the large number of aliquots, an original concentration of target DNA can be determined by using Poisson statistics. The use of digital PCR is particularly advantageous in the case of rare target DNA compared to a background of non-target DNA. The concentration of such rare target DNA can be reliably determined if the number of aliquots used for the digital PCR is large enough.

In order to analyze such a large number of aliquots, it is known to analyze an image showing the partitions with the respective aliquot inside each partition after the thermal treatment. Only in those partitions where target DNA was present, a distinct optical characteristic appears, like a fluorescence signal, a predefined color or the like. In order to simplify the analysis of the image and to enable its automation, usually a grid structure of partitions with the respective aliquots such as the grid structure described in US 2021/0379593 A1 is used. It is furthermore known to use a reference dye generating a signal in a reference light channel in all partitions to localize the partitions within the image or within the respective partition image data. After localizing the partitions, the distinct optical characteristic related to the target DNA is analyzed in a further light channel.

It is an object of the invention to provide a method with an improved analysis of the partition image data, in particular with an improved and reliable detection of the partitions within the partition image data.

SUMMARY OF THE INVENTION

According to a first aspect of the invention, a method for providing an analysis of partition image data, in particular for providing an analysis of a plurality of partition states of at least one microfluidic well within a digital polymerase chain reaction analysis is provided. The method is comprising the following steps:

- receiving the partition image data which is indicating an optical appearance of a plurality of partitions that are arranged in a preexisting grid structure;
- calculating a matrix correlation function R(x,y) for each point (x,y) of the at least one microfluidic well depicted within the partition image data, wherein the matrix correlation function R(x,y) indicates a correlation between a predefined template partition matrix T(x',y') and the at least one depicted microfluidic well;
- calculating a set of assumed grid point locations within the partition image data based on a predefined number of expected grid points and on the calculated matrix correlation function R(x,y);
- detecting a respective partition state based on an optical appearance of the calculated set of assumed grid point locations within the partition image data for each partition of the at least one microfluidic well; and
- providing analysis information based on the detected partition states.

The method according to the first aspect of the invention provides an improved detection of partitions within the partition image data. This allows a particularly reliable detection of the partition states. The invention is based on the finding that even a small misalignment between real grid point location and assumed grid point location might lead to a large number of not correctly analyzed partitions and thus to a false result of the analysis of the partition image data. Furthermore, the invention is based on the fact that all optical media show dispersion, i.e. the index of refraction depends on the spectrum wavelength. Thus, using a reference dye is not only expensive but also leads to a chromatic aberration between both light channels, i.e., the reference light channel and the analyzed light channel, that requires compensation in order to provide a reliable result of the image analysis.

According to the first aspect of the invention, the predefined template partition matrix is used to calculate assumed grid point locations and thereby the location and orientation of the preexisting grid structure. Such an analytical approach advantageously circumvents the need for a reference dye.

The partition image data can be provided by any kind of optical appliance. In case of an dPCR analysis, the partition image data preferably indicates the optical appearance of the plurality of partitions after the thermal treatment. Thereby the partition image data indicates which partitions contain target DNA due to a difference within the optical appearance between partitions with target DNA and partitions without target DNA.

The partition image data according to the invention indicates at least an intensity of a certain wavelength for each point (x,y) of an image of the microfluidic well. A predefined resolution of that image may depend on the optical appliance used to provide the partition image data. The resolution of the partition image data thereby defines the resolution of the predefined template partition matrix T(x',y'). Preferably the predefined template partition matrix is a template for the optical appearance, especially an intensity distribution, of at least one partition. By indicating intensities and/or intensity distributions, respective values x, y, x', y' can be used for calculation steps and thereby allow the calculation of the matrix correlation function R(x,y) for respective points. Thereby the method allows a purely numerical analysis of the partition image data in order to provide the analysis information.

By using the predefined template partition matrix T(x',y'), subsets of the partition image data can be taken into account separately. Preferably, the predefined template partition matrix T(x',y') shows an approximate size of a single partition. The use of the template partition matrix T(x',y') advantageously allows a dust removal. Areas of the partition image data that show a lower intensity due to dust can contribute to the matrix correlation function R(x,y) with the same proportion as areas that show a higher intensity. Thereby large dust artefacts within the partition image data can be suppressed. Details about possible embodiments of such a calculation are described later in the description.

Calculating the set of assumed grid point locations can support a normalization of the received partition image data, enabling further processing steps that form embodiments of the method according to the first aspect. Therefore, the described method can form an advantageous basis for further processing steps as will be apparent in view of the described embodiments.

The predefined number of expected grid points might be larger than the actual number of grid points within the microfluidic device. Thereby, high values of R(x,y) around the actual grid point are taken into account. It is clear to somebody skilled in the art that details of the provided calculation scheme might contain factors that can be found by empirical studies, i.e. after a large number of calculations of the assumed grid point locations. Such details are shown in the course of the detailed description of the embodiments.

The method according to the first aspect forms a part of a superordinate method to analyze fluid within the microfluidic well with its partitions. The superordinate method defines for instance the further processing of the analysis information.

The microfluidic well according to this invention defines a plurality of partitions that are arranged in the preexisting grid structure. The partition image data might further comprise information related to one or more further microfluidic wells. As obvious to someone skilled in the art, the method according to the first aspect of the invention can also be carried out for partition image data that indicates the optical appearance of a plurality of microfluidic wells.

In the following, embodiments of the method according to the first aspect of the invention will be described.

Preferably, in a further step of the method according to the first aspect of the invention, the assumed grid point locations form an assumed grid structure that is further adapted in order to provide a rotational rectification. Due to mechanical tolerances and physical effects, such as a possible focus adjustment while collecting the partition image data, such an additional rotational rectification can become necessary. Therefore, the rotational rectification according to this embodiment supports the reliability of the method according to the first aspect of the invention. The following embodiments and the related variants will show advantageous ways to provide such a rotational rectification numerically.

In a preferred embodiment of the method according to the first aspect of the invention, this method is further comprising the following steps:

- providing filtered partition image data based on the calculated set of assumed grid point locations;
- calculating a row pixel histogram, wherein non-zero pixels of each row within the filtered partition image data are summed up, in order to provide rotated filtered partition image data comprising a rotational rectification of the filtered partition image data, wherein the detection of the respective partition states is further based on the rotated filtered partition image data.

In this embodiment, a rotation of the partition image data compared to the actual orientation of the grid structure might be rectified. This is particularly advantageous in view of a possible focus adjustment or a mechanical movement of the optical appliance providing the partition image data. Non-zero pixels induce a certain light intensity at the respective location of the pixel within the microfluidic well. In a preferred example of this variant, non-zero pixels show a partition with a known optical characteristic, such as a certain color and/or a fluorescence, that leads to a non-zero intensity of light of the studied wavelength, i.e., the studied light channel.

A row pixel histogram is also a line pixel histogram if an orientation is turned. That is why a line pixel histogram is mentioned in the following only as a further histogram in addition to an already existing row pixel histogram. It is obvious that every embodiment involving a row pixel histogram can be realized with a line pixel histogram, too. Every orientation within the embodiments should be defined as the orientation where the used pixel histogram is a row pixel histogram. Thereby a single line pixel histogram is to be regarded as a row pixel histogram within the described framework.

In a variant of the aforementioned embodiment, the rotational rectification of the filtered partition image data is provided by a plurality of calculated row pixel histograms, wherein each calculated row pixel histogram represents a predefined angle of rotation of the filtered partition image data, and wherein a predefined histogram characteristic is analyzed for the plurality of calculated row pixel histograms in order to provide the rotated filtered partition image data. In this variant, different angles of rotation of the filtered partition image data can be studied in order to find the actual angle of rotation. The actual angle of rotation is preferably the angle at which the predefined histogram characteristic shows an extremum, such as a minimum or a maximum value, compared to other angles of rotation.

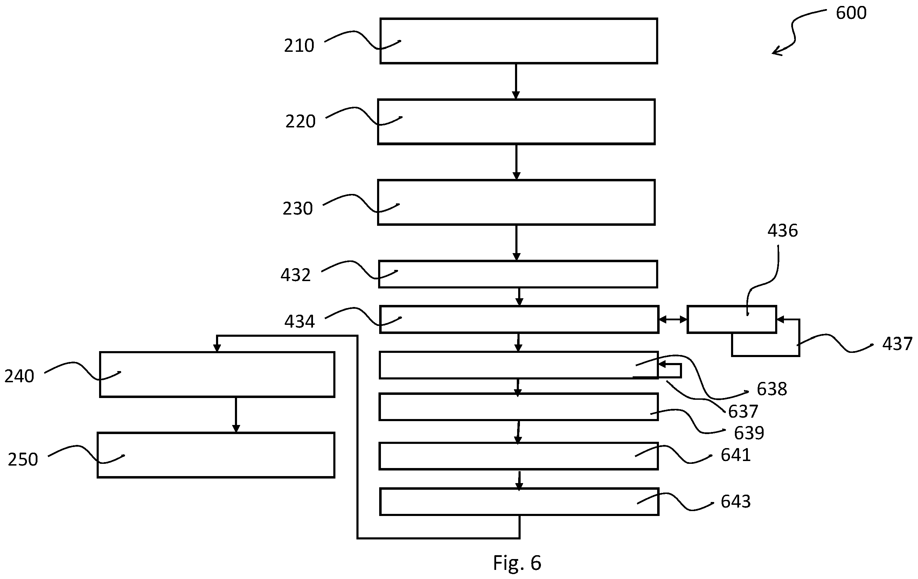

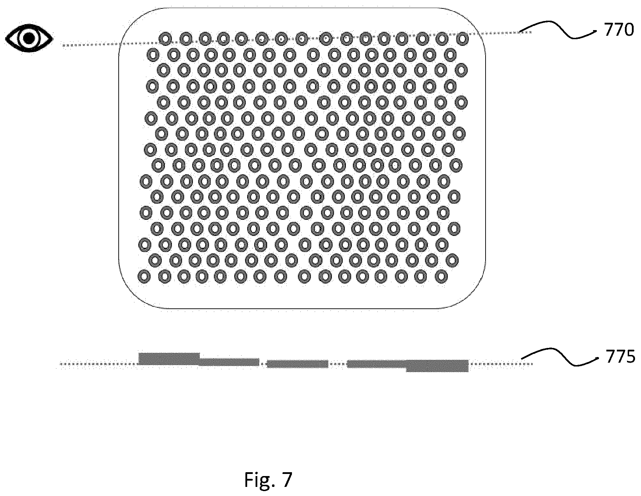

In a preferred example of the aforementioned variant, the rotational rectification comprises two steps, wherein the first step is provided based on the predefined histogram characteristic, and wherein the second step is provided based on a pixel count of non-zero pixels above and/or below an adjustment line that is supposed to be horizontal or vertical according to the first step of the rotational rectification. In this example, the pixel count can provide a further fine tuning of the angle of rotation. The adjustment line forms a threshold with respect to a base of the grid structure of the assumed grid point locations. In that sense the adjustment line can be a numerical tool and not an actual line. Horizontal or vertical according to the first step of the rotational rectification means that it is horizontal or vertical with respect to a base formed by a rectified grid structure after the first step of the rotational rectification. Preferably, both steps of this example are repeated for different angles of rotation in order to fine tune the rotational rectification. The actual angle of rotation might be detected as a local maximum of non-zero pixels. Details of this method will be described in the course of Figs. 6 and 7.

In a further variant of the embodiment introducing the rotational rectification, the method is further comprising the step

- determining a height and/or a width of the at least one depicted microfluidic well based on at least one calculated row pixel histogram.

In this variant, further relevant characteristics of the microfluidic well are determined based on the partition image data. This calculation of the height and/or width may serve as a further security measure that the determined height and width corresponds to the known height and width of the preexisting grid structure. Furthermore, certain partitions are well defined if their position within the height and width of the preexisting grid structure is known. Thereby an alignment between rectified grid structure and the actual preexisting grid structure might be improved.

In a further variant of the embodiment introducing the rotational rectification, the method is further comprising the steps

- calculating a line pixel histogram in a direction perpendicular to the direction of the rows according to the calculated row pixel histogram;
- calculating a corrected set of assumed grid point locations by determining intersections of horizontal and vertical lines within the calculated line pixel histogram and the calculated row pixel histogram.

In this variant, a further numerical step exploits the fact, that the preexisting grid structure is preferably formed by a self-repeating pattern in a horizontal and vertical direction. Therefore, horizontal and vertical lines according to this variant are lines with numerous non-zero pixels and therefore lines with numerous partitions of the preexisting grid structure. Intersections of horizontal and vertical lines therefore define actual grid point locations rather precisely. Thus, the present variant advantageously allows a precise calculation of the corrected set of assumed grid points after the correction with respect to the matrix correlation function R(x',y') and after the rotational rectification according to the embodiment. By using this numerical tool of intersections between peaks of histograms, even the location of those partitions can be found which are not shown in the partition image data due to dust or the like. Therefore, the method according to this variant can reduce the impact of dust on the provided image analysis.

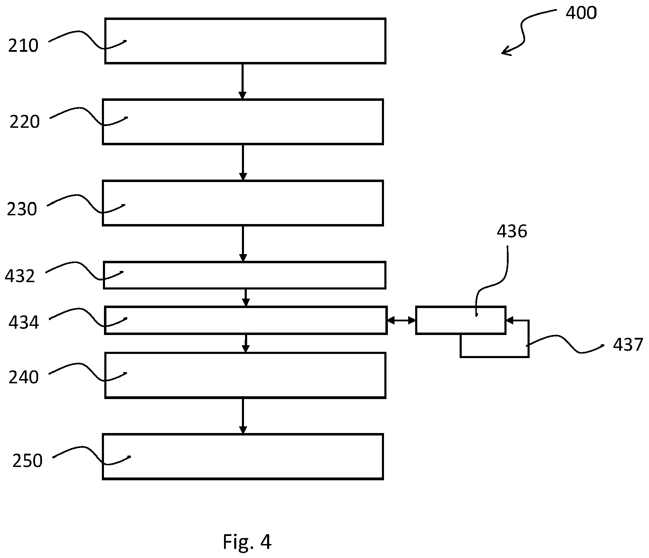

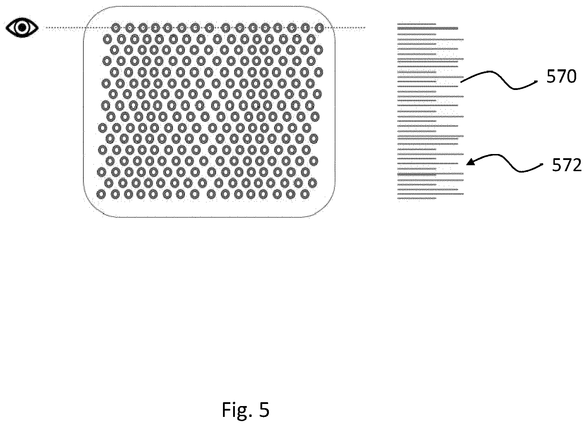

In a preferred variant of the embodiment introducing the rotational rectification, the predefined histogram characteristic comprises a sum of absolute differences between adjacent points within the respective histogram. This predefined histogram characteristic shows if the histogram appears to be spikey, i.e. if there are large differences between adjacent points within the respective histogram. A large value indicates a comb-like structure. Such a structure indicates a little error with respect to rotation between the calculated assumed grid point location and the actual grid point location within the microfluidic well. This kind of predefined histogram characteristic is suitable as histogram characteristic for all the aforementioned variants. Particularly, it allows a very precise calculation of the amount of rotational rectification that is necessary to calculate the rotated filtered partition image data reliably. In particular, this histogram characteristic may help to remove dust related artefacts since single false pixel evaluations do not influence this histogram characteristic very much. Possible examples of the histogram characteristic according to this variant are presented in the course of Figs. 4 and 5.

In a further embodiment of the method, the calculation of the set of assumed grid point locations within the partition image data is based on the matrix correlation function R(x,y), wherein a dynamic threshold is adapted until a number of points (x,y) of the at least one depicted microfluidic well for which the product exceeds the dynamic threshold equals the predefined number of expected grid points. The dynamic threshold is preferably a threshold for R(x,y). Thereby points (x,y) with R(x,y) above the dynamic threshold are counted in order to count the number of grid points for the current threshold value. The use of a dynamic threshold advantageously allows the calculation of the assumed grid point locations in a way that ensures a reasonably limited number of such assumed grid point locations and thereby avoids numerical mistakes that lead to obviously wrong grid point numbers. The calculation according to this embodiment allows an improved automation of the present method according to the first aspect of the invention.

In a particularly preferred embodiment of the present method, the steps for providing an analysis of partition image data are repeated for at least one further light channel within the partition image data. Using multiple light channels according to this invention might be advantageous for chemical reasons in order to reliably detect the respective partition state for each partition. This embodiment is particularly advantageous if different wavelengths of light have to be detected, for instance of a reference dye is used to further optically distinguish partitions with the target DNA from partitions without the target DNA.

In a preferred variant of the aforementioned embodiment, the results of the repeated steps for the at least two light channels within the partition image data are combined to provide an aligned set of assumed grid point locations for detecting the respective partition state for each partition of the at least one microfluidic well. Repeating the present method according to this variant allows an advantageous alignment of the assumed grid point locations for different light channels. This provides a very reliable analysis of the partition image data.

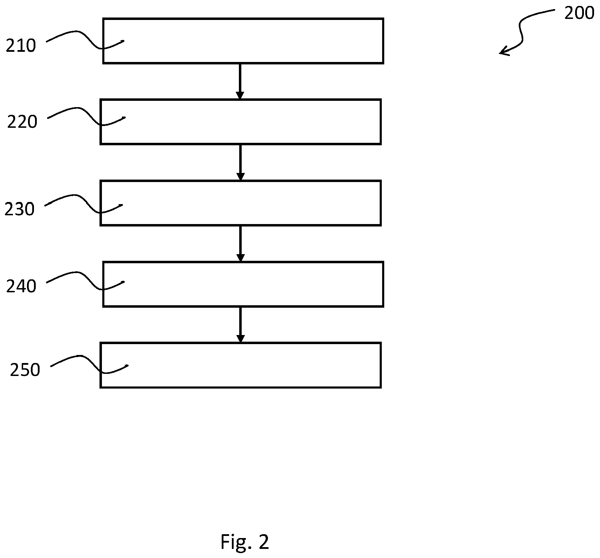

In a further embodiment of the method according to the first aspect of the invention, the predefined template partition matrix T(x',y') essentially describes an expected optical appearance of a single partition within the microfluidic well. The template partition matrix T(x',y') according to this embodiment allows the method to crop out numerically every part of the partition image data except of the region of a single partition. By repeatedly analyzing such regions of a single partition, the resulting matrix correlation function can provide a measure of probability indicating if a certain pixel forms a part of a partition or not. Details of such an analysis are described in the course of Fig. 2.

In a further embodiment of the method, the predefined template partition matrix T(x',y') is a square matrix, in particular at least a 5x5 matrix, preferably at least a 7x7 matrix. Such a rather small template partition matrix, compared to the number of pixels indicated within the partition image data, allows rather fast numerical calculations in the context of this method. Thereby the method may lead to a fast result for providing the analysis information.

According to a second aspect of the invention, a system for providing a molecular analysis of a fluid sample, in particular for providing a digital polymerase chain reaction analysis, is provided. The system is comprising a control unit which is arranged and configured to carry out the method according to at least one of the preceding embodiments.

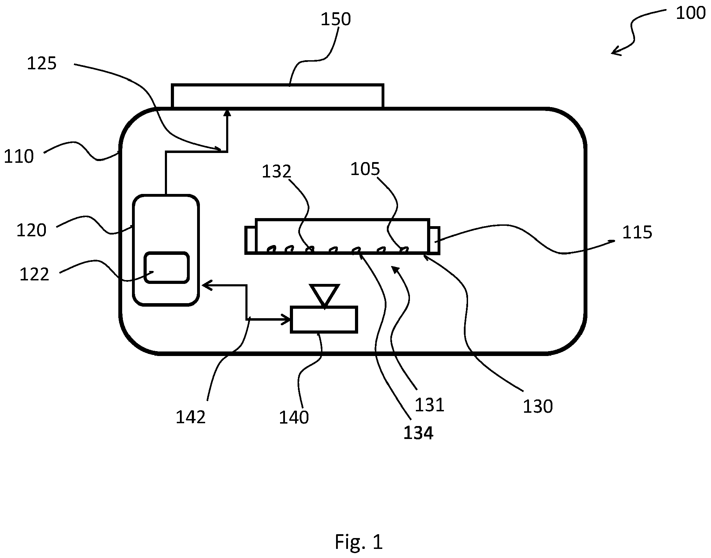

The system according to the second aspect of the invention shares the advantages of the method according to the first aspect of the invention. It is obvious to somebody skilled in the art that the system might provide means to hold the microfluidic well and/or means, such as optical means, to provide the partition image data. Such embodiments of the system are obvious and will not discussed in further detail in the following. One out of numerous possible embodiments of such a system according to the second aspect of the invention is described in the context of Fig.1.

All parts of the system can be arranged in a single housing or can be at least partly spatially separated.

In a preferred embodiment of the system according to the second aspect of the invention, the analyzed reactions are tagged with fluorophores in order to provide a fluorescence image within the partition image data. Such a tagging can improve the recognition of target DNA and/or non-target DNA within the partitions applying the method according to the first aspect of the invention. Alternatively or additionally, the tagging can improve a recognition of partition locations to provide a reliably analysis of the grid structure. The tagging with fluorophores is preferably provided before the respective sample fluid is brought into the microfluidic well, i.e. before the first step of the method according to the first aspect of the invention is performed. In a preferred variant of this embodiment, the relative fluorescence units (RFU) of a predefined range, such as a range between 5 and 10, are amplified to improve the method according to the first aspect of the invention. Thereby, a predefined range of RFUs can be reliably detected and used for further calculations.

According to a third aspect of the invention, a computer program for operating a system comprising program code means for causing a computer to carry out a method according to the first aspect of the invention is provided.

The computer which comprises the computer program may for instance form an integrated part of a microfluidic analysis apparatus of the system according to the second aspect of the invention and be implemented as a microcontroller or microprocessor. In another embodiment, the computer forms an integrated part of a control unit that is spatially separated from the microfluidic analysis apparatus of the system and configured to control this microfluidic analysis apparatus and/or a number of further microfluidic analysis apparatuses.

It shall be understood that the method for providing an analysis of partition image data according to the first aspect of the invention, the system for providing a molecular analysis of a fluid sample according to the second aspect of the invention, and the computer program for operating a system according to the third aspect of the invention have similar or identical embodiments.

These and other aspects of the invention will be apparent from and elucidated with reference to the embodiments described hereinafter.

BRIEF DESCRIPTION OF THE DRAWINGS

In the following drawings:

Fig. 1 shows a first embodiment of a system according to a second aspect of the invention;
Fig. 2 shows a flow diagram of a first embodiment of a method according to a first aspect of the invention;
Fig. 3 shows an example of a set of assumed grid point locations received by the method according to the first embodiment given in Fig. 2;
Fig. 4 shows a flow diagram of a second embodiment of the method according to the first aspect of the invention;
Fig. 5 shows a schematic illustration of a calculation of a row pixel histogram for providing a rotational rectification according to the second embodiment given in Fig. 4;
Fig. 6 shows a flow diagram of a third embodiment of the method according to the first aspect of the invention;
Fig. 7 shows a schematic illustration of a further step of an extended rotational rectification according to the third embodiment of the method;
Fig. 8 shows an example of a corrected set of assumed grid point locations according to the third embodiment of the method.

DETAILED DESCRIPTION OF EMBODIMENTS

Fig. 1 shows a first embodiment of a system 100 according to a second aspect of the invention.

The system 100 is configured for providing a molecular analysis of a fluid sample 105, in particular for providing a digital polymerase chain reaction analysis. It comprises a control unit 120 which is arranged and configured to carry out a method for providing an analysis of partition image data, in particular for providing an analysis of a plurality of partition states of at least one microfluidic well within a digital polymerase chain reaction analysis, according to a first aspect of the invention which is described in the course of the figures 2 to 8.

In order to provide the molecular analysis, the system comprises an analysis apparatus 110 that comprises the control unit 120 and a holding structure 115 for holding a microfluidic device 130. The microfluidic device 130 comprises the fluid sample 105 within a grid structure 131 of single partitions 132 that are structured within microfluidic wells 134. The microfluidic device 130 comprises at least one microfluidic well 134, preferably at least 4 microfluidic wells, in particular at least 10 microfluidic wells. Preferably, the at least one microfluidic well 134 is filled with the fluid sample 105 before the microfluidic device 130 is brought into the analysis apparatus 110 via the holding structure 115. The holding structure 115 in the depicted embodiment is formed by a pair of rails. Alternatively, a moveable holding base, holding arms or the like are used as holding structure in other not shown embodiments of the system.

The system 100 further comprises optical means 140, such as a camera, a CCD-sensors or the like. The optical means 140 are arranged to provide partition image data 142 of at least one microfluidic well 134 of the microfluidic device 130, indicating an optical appearance of a plurality of partitions 132 that are arranged in the preexisting grid structure 131. In the present embodiment, the partition image data 142 is based on an image taken by the optical means 140. The partition image data 142 thereby indicates a light intensity of each pixel of a taken image for light with a predefined wavelength, i.e. for a predefined light channel. The partition image data of a not shown embodiment further indicates the light intensities of the pixels for light with at least one further predefined wavelength, i.e., for a predefined second light channel.

The optical means 140 are arranged and configured to provide the partition image data 142 to a calculation module 122. In the present embodiment, the calculation module 122 forms a part of the control unit 120. In a not shown embodiment, the calculation module is a separate part of the analysis apparatus. In a further not shown embodiment, the calculation module is arranged outside of the analysis apparatus. The calculation module 122 is configured to provide at least some of the steps of the method according to the first aspect of the invention. In particular steps that require calculation are preferably executed by the calculation module 122. Details concerning the method are described in the course of the following embodiments shown in Figs. 2 to 8.

As a last step of said method, analysis information 125 is provided based on detected partition states. The analysis information 125 can be provided to an external device. Additionally or alternatively, the analysis information can be provided to a handheld device and/or to a stationary device of the system according to the second aspect of the invention. In the present embodiment, the analysis information 125 is provided to a user interface 150 of the analysis apparatus 110.

The user interface 150 is arranged and configured to receive the analysis information 125 and to provide a graphical representation indicating the analysis information 125, preferably a concentration of the target DNA studied with the digital PCR analysis. The graphical representation can be a number, a diagram, a color that occurs after a predefined threshold or the like. In another embodiment, the analysis information is provided in order to execute further processing steps.

The optical means 140 of the depicted embodiment are configured to detect a fluorescence image. Before the fluid sample is brought into the microfluidic device 130, fluorophores are brought into the fluid sample in order to tag the analyzed reactions. This enables a reliable detection of partitions within the preexisting grid structure. In this embodiment, the provided partition image data 142 indicates the detected fluorescence image.

Fig. 2 shows a flow diagram of a first embodiment of a method 200 according to a first aspect of the invention.

The method 200 is configured to provide an analysis of partition image data, in particular to provide an analysis of a plurality of partition states of at least one microfluidic well within a digital polymerase chain reaction analysis. The method is preferably executed by a control unit of an analyzing apparatus such as the control unit 120 described for the embodiment in Fig. 1.

The method 200 of the depicted embodiment comprises the steps as described in the following.

A first step 210 comprises a reception of the partition image data which is indicating an optical appearance of a plurality of partitions that are arranged in a preexisting grid structure.

A further step 220 comprises a calculation of a matrix correlation function $R(x,y)$ for each point $(x,y)$ of the at least one microfluidic well depicted within the partition image data, wherein the matrix correlation function indicates a correlation between a predefined template partition matrix $T(x',y')$ and the at least one depicted microfluidic well.

A following step 230 comprises a calculation of a set of assumed grid point locations within the partition image data based on a predefined number of expected grid points and on the calculated matrix correlation function $R(x,y)$. In a not shown embodiment, the calculation of the set of assumed grid point locations is further based on the predefined template partition matrix $T(x', y')$.

A next step 240 comprises a detection of a respective partition state based on an optical appearance of the calculated set of assumed grid point locations within the partition image data for each partition of the at least one microfluidic well.

A final step 250 comprises a providing of analysis information based on the detected partition states.

The steps 210 to 250 of the method 200 are preferably executed by a single control unit 120. The necessary temporal order is given above. The next step within the method 200 needs information that is provided by the respective previous step, so that the order of steps is predetermined. Further steps can be added in order to improve the method 200 as it will be apparent in view of the embodiments given with Figs. 4 and 6.

The predefined template partition matrix $T(x',y')$ preferably indicates the intensity of light from a certain light channel for a single partition that is equal to the partitions provided by the microfluidic well. An example of such a template partition matrix is the following matrix $T(x',y')$:

$$T(x',y')=\begin{pmatrix} 0 & 0 & 0 & 0 & 0 & 0 & 0 \\ 0 & 10 & 20 & 50 & 20 & 10 & 0 \\ 0 & 20 & 50 & 50 & 50 & 20 & 0 \\ 0 & 20 & 100 & 100 & 50 & 20 & 0 \\ 0 & 20 & 100 & 100 & 50 & 20 & 0 \\ 0 & 10 & 20 & 50 & 20 & 10 & 0 \\ 0 & 0 & 0 & 0 & 0 & 0 & 0 \end{pmatrix}$$

The partition has the largest intensity in the center and a vanishing intensity at its periphery.

In a not shown alternative embodiment, the template partition matrix has just 9 entries with non-zero values.

In a further not shown embodiment of the method, the template partition matrix is a 5x5 matrix. In another not shown embodiment, the template partition matrix is larger than a 7x7 matrix. The calculation steps given in the following can generally be performed in an identical way for larger and for smaller template partition matrices. The values used for the template partition matrix can be different for different embodiments of the method according to the first aspect of invention. In a not shown embodiment, the template partition matrix comprises values between 0 and 1 in order to indicate an intensity and/or the optical appearance of a partition of the microfluidic well. Furthermore, the template partition matrix of a further not shown embodiment indicates the optical appearance of more than one partition of the microfluidic well.

Using such a template partition matrix reduces the numerical effort since a calculation with large matrices is avoided.

After defining the template partition matrix T(x',y'), the matrix correlation function R(x,y) can be calculated based on the partition image data I(x,y). The template partition matrix T(x',y') can have different structures. A possible embodiment of this function is formed by the following matrix correlation function R(x,y):

$$R(x,y) = \frac{\sum_{x',y'}(T(x',y') * I(x+x',y+y'))}{\sqrt{\sum_{x',y'} T(x',y')^2 * \sum_{x',y'} I(x+x',y+y')^2}}$$

It can be recognized that the correlation between the template partition matrix T(x',y') and the partition image data I(x,y) is taken into account for the size of the template partition matrix T(x',y') around the pixel (x,y) within the partition image data I(x,y). Other structures of the matrix correlation function R(x,y) can be reached for instance by using another normalization, i.e. another denominator of said fraction, which would lead to similar results for the calculation steps according to this invention.

There exist several calculation schemes for calculating assumed grid point locations in the context of step 230 of the method 200. A possible method is based on an element-wise product of the matrix correlation function R(x,y) and the predefined template partition matrix T(x',y'). A preferred method is formed by applying a dynamic threshold to the matrix correlation function R(x,y), wherein the dynamic threshold is adapted until a number of points (x,y) of the at least one depicted microfluidic well for which the matrix correlation function R(x,y) exceeds the dynamic threshold equals the predefined number of expected grid points. This defines an iterative method that ensures that a number of assumed grid point locations equals the predefined number of expected grid points.

Starting from a threshold that is rather high, as for instance 0.9, the dynamic threshold decreases in steps, such as 0.01-steps or 0.05-steps, until the number of matrix points (x,y) where the matrix correlation function R(x,y) is larger than the threshold reaches at least approximately the predefined number of expected grid points. Preferably, the step size for decreasing the dynamic threshold is chosen small enough that said number essentially equals the number of expected grid points. The predefined number of expected grid points might be larger than the actual number of grid points within the microfluidic device. Thereby, high values of R(x,y) around the actual grid point are taken into account. The predefined number of expected grid points is preferably at least 2 times the actual number of grid points, in particular at least 4 times the actual number of gid points.

It should be clear for somebody skilled in the art that the presented calculation scheme can be adapted by empirical values, like a starting value of the dynamic threshold that empirically tends to lead to a fast iteration or the like. The predefined number of expected grid points can also be a value that shows good results over a large number of calculations empirically.

In a not shown alternative variant, the iterative method is not used for the respective values R(x,y) but for a horizontal and/or vertical line histogram of R(x,y), showing where the grid points can be found vertically and/or horizontally.

The calculation scheme presented in this depicted embodiment first detects partition-like shapes by using the template partition matrix and by using the matrix correlation function. Afterwards a template matching-algorithm is used to find as many partition-like signals as possible within the image partition data. As a result, the number of partition-like signals is reduced by decreasing said threshold until the number of partition-like signals is at least an a range of the predefined number of expected grid points that is known from the known microfluidic well with its grid structure. It is obvious to a person skilled in the art that other known and established template matching-algorithms can be used for the method according to the first aspect of the invention. Respective embodiments of other template matching-algorithms are well-known and not discussed in the following.

As a result of the steps described for the method 200 according to the shown embodiment, larger shapes within the partition image data, such as dusty areas, are removed.

Knowing the partition grid, the classification whether a partition has target DNA or not can be simply executed by summing up values of the partition image data at the calculated grid point. For instance, points of a 7x7 matrix around the calculated grid point can be summed up in order to decide, if the optical appearance indicates the target DNA. Summing such values up leads to the relative fluorescence units (RFU) of the respective partition.

In a not shown embodiment of the method, not trusted partitions can be marked manually and/or automatically in order to provide a further method to study such marked partitions separately.

In a further not shown embodiment, an 8x8 matrix with one pixel at each corner cut out forms the template partition matrix. This can be advantageous since this compensates 1 pixel jitter that may exists due to image resolution in the context of a physical grid granularity alignment error.

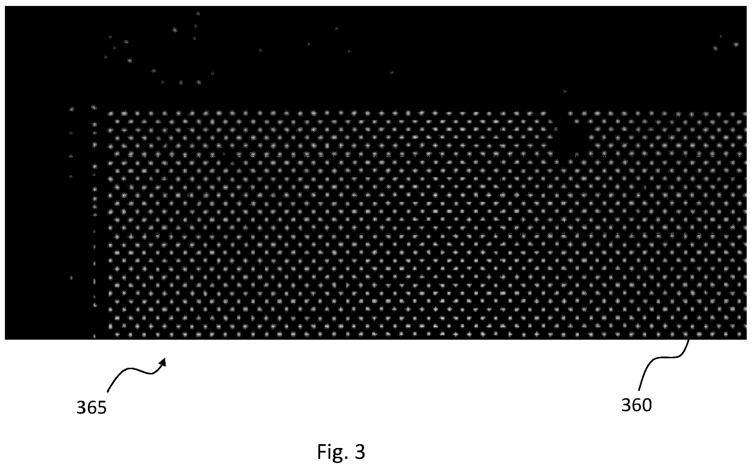

Fig. 3 shows an example of a set of assumed grid point locations 360 provided by the method 200 according to the first embodiment given in Fig. 2.

It is clear from Fig. 3 that differences within the optical appearance between different partitions are not apparent anymore. In that sense, the method 200 leads to a normalization of the partitions. One can recognize the grid structure 365 of the shown part of the microfluidic well while single partitions are removed due to numerical reasons within the calculation scheme.

The original partition image data within this embodiment is not shown since without any filtering the respective image of the microfluidic well just appears to be a black area.

Fig. 3 illustrates that the method 200 according to the first aspect of the invention allows a certain dust removal and provides the assumed grid point locations in a reliable way. In that sense, the presented method finds an assumed grid structure that can also be used for further processing. Based on the calculated assumed grid point locations, the partitions can be reliably detected with their respective partition state within the partition image data. Afterwards, the analysis of the partition states leads to the analysis information.

In a not shown embodiment, the presented steps for providing an analysis of partition image data are repeated for at least one further light channel within the partition image data. That can lead to two slightly different assumed grid point locations and therefore to different assumed grid structures. In a preferred variant of the aforementioned embodiment, results of the repeated steps for the at least two light channels within the partition image data are combined to provide an aligned set of assumed grid point locations for detecting the respective partition state for each partition of the at least one microfluidic well. Thereby a particularly reliable aligned set of assumed grid point locations can be used to detect the partition states within the provided partition image data.

Fig. 4 shows a flow diagram of a second embodiment of the method 400 according to the first aspect of the invention.

The second embodiment forms a preferred embodiment of the present method since it allows a particularly reliable detection of the partition states within the partition image data.

The method 400 comprises all the steps of the method 200 shown in Fig. 2 and additionally the steps as described in the following.

A first additional step 432, preferably executed between step 230 and 240 of the method 200, comprises a providing of filtered partition image data based on the calculated set of assumed grid point locations.

A following additional step 434, preferably executed after the additional step 432, comprises a calculation of a row pixel histogram, wherein non-zero pixels of each row within the filtered partition image data are summed up, in order to provide a rotated filtered partition image data comprising a rotational rectification of the filtered partition image data, wherein the detection of the respective partition states is further based on the rotated filtered partition image data.

By using the row pixel histogram, the preferably self-repeating structure of the microfluidic well is advantageously used to recognize a slight rotational mistake between the assumed set of grid point locations and the actual grid point locations. Such a rotational misalignment can be a result of a mechanical movement of the optical means that provide the partition image data. If a perfect rotational alignment is reached, a comb-like structure of the row pixel histogram would be the expected result, as it can be understood in view of Fig. 5.

The embodiment shown in Fig. 4 comprises a last additional step 436. In a not shown embodiment, only the steps 432 and 434 are added to method 200. The particularly advantageous step 436 forms a substep of step 434. According to step 436, the rotational rectification of the filtered partition image data is provided by a plurality of calculated row pixel histograms, wherein each calculated row pixel histogram represents a predefined angle of rotation of the filtered partition image data, and wherein a predefined histogram characteristic is analyzed for the plurality of calculated row pixel histograms in order to provide the rotated filtered partition image data. The repetition of this step for different predefined angles of rotation is indicated in Fig. 4 by the repetition arrow 437.

By studying different angles of rotation, the angle with the most perfect alignment between assumed grit point location and actual grid point location may be found advantageously using the predefined histogram characteristic. The different angles preferably show an angle step size of less than 0.5°, in particular of less than 0.2°, as for instance of less than 0.1°. A small angle step size can lead to a particularly reliable analysis.

The predefined histogram characteristic preferably comprises a sum of absolute differences between adjacent points within the histogram. An example of such a histogram characteristic C is given in the following:

$$C = \sum_{y-1} \left( abs \left( L_{y+1} - L_y \right) \right)$$

This histogram characteristic indicates which row pixel histogram shows a comb-like structure. If there are some spikes, the given histogram characteristic C will have a larger value as in the case of a less spiky pattern. Fig. 5 visualizes such a comb-like, i.e. spiky, structure of the row pixel histogram. If an angle of rotation is found with a maximal value of C, the row pixel histogram has a comb-like structure meaning that a horizontal axis of the grid structure can be defined and the rotational rectification has been provided.

Other histogram characteristic for studying the necessary rotational rectification are used in further not shown embodiment of the method. Preferably such alternative histogram characteristics also provide a measure for the spikiness of the row pixel histogram.

Fig. 5 shows a schematic illustration of a calculation of a row pixel histogram 570 for providing a rotational rectification according to the second embodiment given in Fig. 4.

The row pixel histogram 570 shows said comb-like structure meaning that this row pixel histogram has been reached at the end of step 436 of method 400. After a number of repetitions for different angles of rotation, the depicted row pixel histogram 570 has been reached for the angle of rotation with the largest value for the histogram characteristic C as given above.

Fig. 5 illustrates that non-zero pixels of each row within the filtered partition image data are summed up in order to analyze the row pixel histogram 570. The eye in Fig. 5 further illustrates the orientation of a respective row that defines an axis for summing up the non-zero pixels of that axis. Fig. 5 illustrates furthermore that the absolute height of the analyzed grid structure might be calculated by studying the row pixel histogram. There es the comb-like region 572 within the row pixel histogram 570. That comb-like region 572 will disappear at the borders of the grid structure and thereby show the height of the grid structure.

The steps discussed for the method 400 containing the row pixel histogram can also alternatively or additionally be provided by a line pixel histogram, counting the non-zero pixels of each column. The discussed steps can be executed analogously to the row pixel histogram. Such a line pixel histogram may lead to a value for the width of the grid structure by studying the comb-like region of the line pixel histogram that will be apparent after the rotational rectification.

In a further embodiment, a row pixel histogram and a line pixel histogram are studied in parallel or in sequence and width and height of the grid structure are calculated as well. After calculating the row pixel histogram and the line pixel histogram, both histograms can be used to calculate a corrected set of assumed grid point locations by determining intersections of horizontal and vertical lines within the calculated line pixel histogram and the calculated row pixel histogram. This will also be described for the third embodiment of the method shown in Fig. 6.

Even if single partitions were not taken into account within the assumed set of grid point locations, e.g. because of dust or the like, the corrected set of assumed grid point locations can provide all partition locations. Thus, the method 400 can completely extract the impact of dust out of the analyzed data. This will also be shown in the course of the method 600 described in Figs. 6 to 8.

If multiple light channels are analyzed, such a corrected set of grid point locations can be determined for every light channel so that all channel grids can be transformed to become aligned with one of the grids that is used as reference channel grid.

Fig. 6 shows a flow diagram of a third embodiment of the method 600 according to the first aspect of the invention.

The method 600 comprises all steps already discussed for the method 400 shown in Fig. 4 and a further balancing step 638 that is preferably provided after the steps 432 and 434 and before step 240.

The balancing step 638 is based on a pixel count of non-zero pixels above and/or below an adjustment line that is supposed to be horizontal or vertical according to the previous steps of the rotational rectification. The balancing step 636 will be explained in more detail in the course of Fig. 7.

As a result of that calculation scheme, the rotational rectification comprises a first part based on the predefined histogram characteristics and a second part based on the balancing step 638. In the first part, a suitable angle of rotation is reached by trying different angles of rotation and calculating the predefined histogram characteristic. In the second part, the orientation that is supposed to be horizontal or vertical after the first part is adjusted slightly in order to prove if a more suitable angle of rotation can be found. This adjustment is an iterative adjustment which is illustrated by the repetition arrow 637.

After the balancing step 638, a further step 639 is provided comprising a determining of a height and/or a width of the at least one depicted microfluidic well based on at least one calculated row pixel histogram and/or line pixel histogram.

Determining the height and/or width of the grid structure can be done straightforward after calculating the respective histogram. The part of the histogram where the comb-like structure ends is also the end of the analyzed grid structure.

In the depicted embodiment of the method 600, two further steps are additionally provided.

A first additional step 641 comprises a calculation of a line pixel histogram in a direction perpendicular to the direction of the rows according to the calculated row pixel histogram.

A subsequent step 643 comprises a calculation of a corrected set of assumed grid point locations by determining intersections of horizontal and vertical lines within the calculated line pixel histogram and the calculated row pixel histogram.

These steps 641, 643 are executed after the balancing step 638. In a not shown embodiment, these steps are executed in a method without the balancing step.

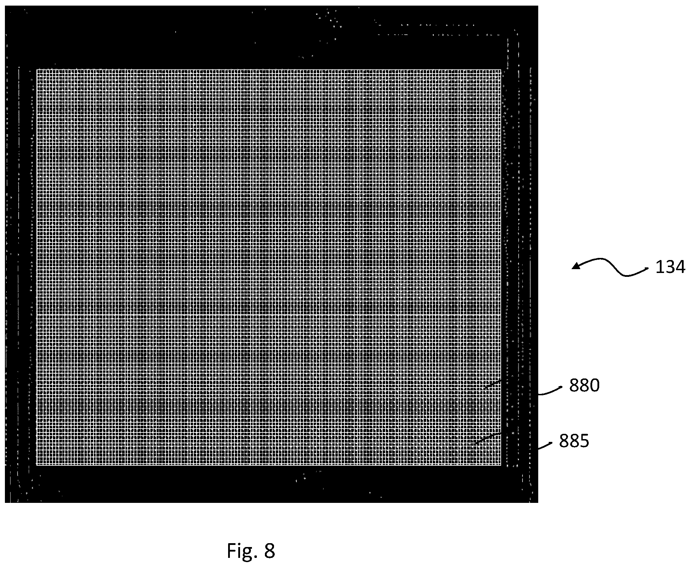

The corrected set of assumed grid points is a numerical result that takes into account the self-repeating structure of the preexisting grid of the microfluidic well. In that sense, even the location of partitions that can hardly be recognized within the partition image data can be used for the analysis of partition states. An example of such a corrected set of assumed grid point locations is shown in Fig.8.

Fig. 7 shows a schematic illustration of a further step of an extended rotational rectification according to the third embodiment of the method 600.

The schematic illustration shows the balancing step 638. The eye symbolizes the orientation of the adjustment line 770 that is supposed to be horizontal after the first part of the rotational rectification. Starting from that adjustment line 770 a pixel distribution 775 of non-zero pixels above and/or below the line left and right from the center is used to adjust the rotation so that the best equilibrium is reached in a couple of small iterations around the center.

The diagram below illustrates the pixel distribution for a certain adjustment line 770.

The balancing step 638 further improves the accuracy of the rotational rectification. Therefore, further analysis, such as the determination of height and/or weight of the grid structure, is preferably performed after the balancing step.

Fig. 8 shows an example of a corrected set of assumed grid point locations 880 according to the third embodiment of the method.

As described above, a combination of a row pixel histogram and a line pixel histogram can be advantageously used to calculate the corrected set of assumed grid point locations 880 as intersections of respective lines and rows within the respective histogram.

The assumed grid point locations are respective points 885 within the microfluidic well 134. The location of the microfluidic well 134 is defined by the calculated width and height. In order to detect the partition states in step 240, the points 885 of the assumed grid point locations have to be checked subsequently with respect to their optical appearance.

As a result, the method 600 leads to a very reliable analysis of the partition image data. Dust artefacts of large areas and of single pixels can be removed. Blistering artefacts can be removed. Leaks in the partition image data can be overcome by the corrected set of assumed grid point locations.

The described embodiments of the method according to the first aspect of the invention are particularly advantageous for analyzing more than one light channel by providing a calculation scheme to ensure an alignment between the resulting assumed grid structures.

While the present invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure and the appended claims. In particular the invention is not restricted to its use within digital PCR.

In the claims, the word "comprising" does not exclude other elements or steps, and the indefinite article "a" or "an" does not exclude a plurality.

A single step or other units may fulfill the functions of several items recited in the claims. The mere fact that certain measures are recited in mutually different dependent claims does not indicate that a combination of these measures cannot be used to advantage.

Any reference signs in the claims should not be constructed as limiting the scope.

LIST OF REFERENCE SIGNS

| | |
|---|---|
| 100 | system |
| 105 | fluid sample |
| 110 | analysis apparatus |
| 115 | holding structure |
| 120 | control unit |
| 122 | calculation module |
| 130 | microfluidic device |
| 131 | grid structure |
| 132 | partition |
| 134 | microfluidic well |
| 140 | optical means |
| 142 | partition image data |
| 125 | analysis information |
| 150 | user interface |
| 200, 400, 600 | method |
| 210, 220, 230, 240 | method steps |
| 250, 432, 434, 436 638, 639, 641, 643 360 | set of assumed grid point locations |

| 365 | grid structure |
| 437, 637 | repetition arrow |
| 570 | row pixel histogram |
| 572 | comb-like region |
| 770 | adjustment line |
| 775 | pixel distribution |
| 880 | corrected set of assumed grid point locations |
| 885 | point |

**Claims**

1.  A method (200) for providing an analysis of partition image data (142), in particular for providing an analysis of a plurality of partition states of at least one microfluidic well (134) within a digital polymerase chain reaction analysis, the method (200) comprising

    - receiving the partition image data (142) which is indicating an optical appearance of a plurality of partitions (132) that are arranged in a preexisting grid structure (365);
    - calculating a matrix correlation function $R(x,y)$ for each point $(x,y)$ of the at least one microfluidic well (134) depicted within the partition image data (142), wherein the matrix correlation function $R(x,y)$ indicates a correlation between a predefined template partition matrix $T(x',y')$ and the at least one depicted microfluidic well (134);
    - calculating a set of assumed grid point locations (360) within the partition image data (142) based on a predefined number of expected grid points and on the calculated matrix correlation function $R(x,y)$;
    - detecting a respective partition state based on an optical appearance of the calculated set of assumed grid point locations (360) within the partition image data (142) for each partition (132) of the at least one microfluidic well (134); and
    - providing analysis information (125) based on the detected partition states.

2.  The method (400) according to claim 1, further comprising

    - providing filtered partition image data based on the calculated set of assumed grid point locations (360);
    - calculating a row pixel histogram (570), wherein non-zero pixels of each row within the filtered partition image data are summed up, in order to provide a rotated filtered partition image data comprising a rotational rectification of the filtered partition image data, wherein the detection of the respective partition states is further based on the rotated filtered partition image data.

3.  The method (400) according to claim 2, wherein the rotational rectification of the filtered partition image data is provided by a plurality of calculated row pixel histograms (570), wherein each calculated row pixel histogram (570) represents a predefined angle of rotation of the filtered partition image data, and wherein a predefined histogram characteristic is analyzed for the plurality of calculated row pixel histograms (570) in order to provide the rotated filtered partition image data.

4.  The method (600) according to claim 3, wherein the rotational rectification comprises two steps, wherein the first step is provided based on the predefined histogram characteristic, and wherein the second step is provided based on a pixel count of non-zero pixels above and/or below an adjustment line (770) that is supposed to be horizontal or vertical according to the first step of the rotational rectification.

5.  The method (600) according to at least one of claims 2 to 4, further comprising

    - determining a height and/or a width of the at least one depicted microfluidic well (134) based on at least one calculated row pixel histogram (570).

6.  The method (600) according to at least one of claims 2 to 5, further comprising

    - calculating a line pixel histogram in a direction perpendicular to the direction of the rows according to the calculated row pixel histogram (570);
    - calculating a corrected set of assumed grid point locations (880) by determining intersections of horizontal and vertical lines within the calculated line pixel histogram and the calculated row pixel histogram (570).

7. The method (400) according to at least one of claims 3 to 6, wherein the predefined histogram characteristic comprises a sum of absolute differences between adjacent points within the histogram.

8. The method (200) according to at least one of the preceding claims, wherein the calculation of the set of assumed grid point locations (360) within the partition image data (142) is based on the matrix correlation function R(x,y), wherein a dynamic threshold is adapted until a number of points (x,y) of the at least one depicted microfluidic well (134) for which the matrix correlation function R(x,y) exceeds the dynamic threshold equals the predefined number of expected grid points.

9. The method (200) according to at least one of the preceding claims, wherein the steps for providing an analysis of partition image data (142) are repeated for at least one further light channel within the partition image data (142).

10. The method (200) according to claim 9, wherein results of the repeated steps for the at least two light channels within the partition image data (142) are combined to provide an aligned set of assumed grid point locations (360) for detecting the respective partition state for each partition of the at least one microfluidic well (134).

11. The method (200) according to at least one of the preceding claims, wherein the predefined template partition matrix T(x',y') essentially describes an expected optical appearance of a single partition (132) within the microfluidic well (134).

12. The method (200) according to at least one of the preceding claims, wherein the predefined template partition matrix T(x',y') is a square matrix, in particular at least a 5x5 matrix, preferably at least a 7x7 matrix.

13. A system (100) for providing a molecular analysis of a fluid sample (105), in particular for providing a digital polymerase chain reaction analysis, comprising a control unit (120) which is arranged and configured to carry out the method (200) according to at least one of the preceding claims.

14. The system (100) according to claim 13, wherein the analyzed reactions are tagged with fluorophores in order to provide a fluorescence image within the partition image data (142).

15. A computer program for operating a system comprising program code means for causing a computer to carry out a method (200) according to at least one of the claims 1 to 12.

Fig. 1

EP 4 435 717 A1

200

210

220

230

240

250

Fig. 2

Fig. 3

Fig. 4

Fig. 5

Fig. 6

Fig. 7

Fig. 8

Europäisches
Patentamt

European
Patent Office

Office européen
des brevets

# EUROPEAN SEARCH REPORT

Application Number

EP 23 16 4102

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 976 434 B1 (LIFE TECHNOLOGIES CORP [US]) 2 August 2017 (2017-08-02) | 1,8-15 | INV. |
| Y | * paragraphs [0007], [0010] - [0012], [0026] - [0029], [0039] - [0041], [0043] - [0044], [0060] - [0061]; figures 3, 5A * | 2-7 | G06T7/11 G06T7/73 G16B40/10 G06T7/00 |
| X | CHARALAMBOUS CHRISTOFOROS C ET AL: "A new method for gridding DNA microarrays", COMPUTERS IN BIOLOGY AND MEDICINE, NEW YORK, NY, US, vol. 43, no. 10, 2 July 2013 (2013-07-02), pages 1303-1312, XP028715734, ISSN: 0010-4825, DOI: 10.1016/J.COMPBIOMED.2013.06.005 * Section 2.4 Spots detection * | 1,13,15 | |
| Y | JP 2009 204414 A (CANON KK) 10 September 2009 (2009-09-10) * paragraph [0047] * | 2-7 | |
| | | | TECHNICAL FIELDS SEARCHED (IPC) |
| Y | US 2005/244040 A1 (LI XINGYUAN [US] ET AL) 3 November 2005 (2005-11-03) * paragraphs [0021] - [0028], [0079] - [0080] * | 2-7 | G06F G06T G16B |
| A | KACHOUIE N ET AL: "Arraycount, an algorithm for automatic cell counting in microwell arrays", BIOTECHNIQUES, INFORMA HEALTHCARE, US, vol. 47, no. 3, Suppl. S, 1 September 2009 (2009-09-01), pages IX-XV, XP002697422, ISSN: 0736-6205, DOI: 10.2144/000113202 * Section: Well Detection * | 1,13,15 | |
| A | EP 3 043 319 A1 (COMPLETE GENOMICS INC [US]) 13 July 2016 (2016-07-13) * paragraph [0055] - paragraph [0083] * | 1,13,15 | |

The present search report has been drawn up for all claims

| Place of search | Date of completion of the search | Examiner |
|---|---|---|
| Munich | 16 June 2023 | Tibrewal-Fabricius |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 16 4102

This annex lists the patent family members relating to the patent documents cited in the above-mentioned European search report.
The members are as contained in the European Patent Office EDP file on
The European Patent Office is in no way liable for these particulars which are merely given for the purpose of information.

16-06-2023

| Patent document cited in search report | | Publication date | Patent family member(s) | | Publication date |
|---|---|---|---|---|---|
| EP 2976434 | B1 | 02-08-2017 | EP | 2976434 A1 | 27-01-2016 |
| | | | US | 2016275687 A1 | 22-09-2016 |
| | | | US | 2018276849 A1 | 27-09-2018 |
| | | | WO | 2014153369 A1 | 25-09-2014 |
| JP 2009204414 | A | 10-09-2009 | NONE | | |
| US 2005244040 | A1 | 03-11-2005 | US | 2005244040 A1 | 03-11-2005 |
| | | | WO | 2005109316 A2 | 17-11-2005 |
| EP 3043319 | A1 | 13-07-2016 | CN | 103025927 A | 03-04-2013 |
| | | | EP | 2563953 A1 | 06-03-2013 |
| | | | EP | 3043319 A1 | 13-07-2016 |
| | | | JP | 5654668 B2 | 14-01-2015 |
| | | | JP | 6042859 B2 | 14-12-2016 |
| | | | JP | 2013527848 A | 04-07-2013 |
| | | | JP | 2015042185 A | 05-03-2015 |
| | | | SG | 184963 A1 | 29-11-2012 |
| | | | US | 2011268347 A1 | 03-11-2011 |
| | | | US | 2015080231 A1 | 19-03-2015 |
| | | | WO | 2011137183 A1 | 03-11-2011 |

EPO FORM P0459

For more details about this annex : see Official Journal of the European Patent Office, No. 12/82

**REFERENCES CITED IN THE DESCRIPTION**

**Patent documents cited in the description**

- US 20210379593 A1